# EUROPÄISCHE PATENTSCHRIFT

(11) **EP 0 244 854 B1**
(45) Veröffentlichungstag und Bekanntmachung des Hinweises auf die Patenterteilung: **28.07.1993**
(21) Anmeldenummer: 87106590.0
(22) Anmeldetag: 07.05.1987
(51) Int. Cl.: A61B 17/10

(54) **Wundklammerzange**
Wound clip forceps
Pince à serre-fine

(30) Priorität: 07.05.1986 DE 3615405
(43) Veröffentlichungstag der Anmeldung: 11.11.1987
(73) Patentinhaber: Diener, Helmut, D-78532 Tuttlingen (DE)
(72) Erfinder: Seifert, Josef, D-7451 Grosselfingen (DE)
(74) Vertreter: Liska, Horst, Dr.-Ing.

(56) Entgegenhaltungen:
- EP-A- 0 040 157
- EP-A- 0 076 744
- WO-A-82/02486

## Beschreibung

Die Erfindung betrifft eine Wundklammerzange mit einem Zangenhauptteil, mit einem an dem Zangenhauptteil gehaltenen Magazin, welches zwei Profilteile, die gemeinsam einen langgestreckten, im Querschnitt im wesentlichen U-förmigen Führungsschacht für einen Vorrat von im wesentlichen U-förmigen Wundklammern bilden, einen in dem Führungsschacht verschiebbaren Schieber sowie eine den Schieber auf eine Klammerabgabeöffnung des Führungsschachts zu vorspannende Klammervorschubfeder aufweist, mit vor der Klammerabgabeöffnung angeordneten, den Klammerausschubweg begrenzenden Anschlagmitteln, mit einem Klammerbiegemechanismus, welcher einen im Bereich der Klammerabgabeöffnung des Magazins angeordneten Amboß und einen relativ zu dem Amboß beweglich an dem Zangenhauptteil geführten Biegestempel aufweist und mit einem schwenkbar an dem Zangenhauptteil gelagerten Zangengriffteil zur Betätigung des Klammerbiegemechanismus.

Eine Wundklammerzange dieser Art ist aus der EP-A-142 225 bekannt. Bei dieser Wundklammerzange ist nicht nur der Biegestempel sondern auch der Amboß an dem Zangenhauptteil gehalten. Der Amboß bildet den vor der Klammerabgabeöffnung des Magazins das ungewollte Ausschieben der Wundklammern verhindernden Anschlag. Das Magazin ist nicht auswechselbar. Dies hat zur Folge, daß die Wundklammerzange während der Operation nicht nachgeladen werden kann, falls der Klammervorrat zu Ende gehen sollte. Es muß eine vollständige zweite Wundklammerzange zur Verfügung stehen, mit der Folge, daß nach der Operation mehrere vollständige Wundklammerzangen sterilisiert werden müssen. Darüberhinaus ist die bekannte Wundklammerzange konstruktiv vergleichsweise aufwendig.

Aus WO 82/02486 ist eine Wundklammerzange bekannt, die ähnlich der vorstehend erläuterten Wundklammerzange ein Zangenhauptteil und ein an dem Zangenhauptteil gehaltenes Magazin umfaßt. Das Magazin hat zwei Profilteile, die gemeinsam einen langgestreckten, im Querschnitt im wesentlichen U-förmigen Führungsschacht für einen Vorrat von im wesentlichen U-förmigen Wundklammern bilden, und in dem Führungsschacht ist ein von einer Klammervorschubfeder auf eine Klammerabgabeöffnung des Führungsschachts vorgespannter Schieber angeordnet. Das Magazin hat eine Frontplatte, an der vor der Klarnerabgabeöffnung ein als Zange ausgebildeter Biegemechanismus gelagert ist, der die von der Klammervorschubfeder gegen die Frontplatte gedrückte Wundklammer an ihren Schenkeln erfaßt und zu einem Ring biegt. An der Frontplatte des Magazins ist ein Anschlag angeordnet, der verhindert, daß die Wundklammer während des Biegevorgangs in das Magazin hinein ausweicht. Die in dem Biegemechanismus liegende, jeweils vorderste Wundklammer wird ausschließlich durch Reibung in dem Magazin gehalten. Die beiden Profilteile sind durch eine Steckführung aneinander geführt, die das Zusammensetzen der Profilteile quer zur Längsrichtung des Führungsschachts erlaubt.

Es ist ferner bekannt, Wundklammerzangen in Einwegversion herzustellen, die nach Gebrauch weggeworfen werden. Da derartige Wundklammerzangen, obwohl sie für Einwegbenutzung konzipiert sind, vergleichsweise aufwendig sind, ist diese Lösung vergleichsweise teuer.

Es ist Aufgabe der Erfindung, eine konstruktiv einfache Wundklammerzange anzugeben, bei welcher das Magazin betriebsmäßig auswechselbar sein soll, so daß die Wundklammerzange auch während der Operation mit einem neuen Vorrat an Wundklammern nachgeladen werden kann. Darüberhinaus soll sichergestellt sein, daß das Magazin auf einfachste Weise mit den Wundklammern geladen werden kann.

Diese Aufgabe wird ausgehend von der eingangs erläuterten Wundklammerzange erfindungsgemäß dadurch gelöst, daß die beiden Profilteile des Magazins in Längsrichtung des Führungsschachts verlaufende Steckführungen aufweisen, die die Profilteile quer zum Führungsschacht aneinander allseitig fixiert führen, daß die den Klammerausschubweg begrenzenden Anschlagmittel zwei beiderseits der Klammerabgabeöffnung vor dieser an dem Magazin angeordnete Anschläge aufweisen, daß auch der Amboß an dem Magazin angeordnete ist und die Klammerabgabeöffnung den Amboß umgibt und daß das Magazin zusammen mit dem Schieber und der Klammervorschubfeder als Einheit lösbar an dem Zangen hauptteil befestigt ist.

Für die Operation können mehrere mit Wundklammern gefüllte Magazine bereitgehalten werden, die nacheinander an dem Zangenhauptteil der Wundklammerzange befestigt werden. Der vorzugsweise durch zwei beiderseits der Klammerausschuböffnung angeordnete Nasen oder dergleichen gebildete Anschlag verhindert auch bei abgenommenem Magazin, daß die Klammervorschubfeder den Klammervorrat aus dem Magazin ausschiebt. Da die Profilteile in Längsrichtung des Führungsschachts ineinander steckbar sind, läßt sich das Magazin problemlos mit den Wundklammern laden, selbst wenn es sich um lose, d.h. nicht miteinander verbundene Wundklammern handelt. Die Wundklammern können in eines der beiden Profilteile eingelegt werden, und das andere Profilteil kann sukzessive über die bereits eingelegten Wundklammern geschoben werden, wodurch verhindert wird, daß die Wundklammern beim Einlegen wieder herausfallen.

Das Magazin kann als Einwegmagazin ausgebildet sein, wobei zweckmäßigerweise die beiden Profilteile nach dem Einfüllen der Wundklammern dauerhaft miteinander verbunden, beispielsweise verrastet oder verklebt werden. Das Magazin eignet sich jedoch auch vorzüglich zum Nachladen. Insbesondere kann es in zerlegtem oder zumindest teilweise geöffnetem Zustand leicht und sicher sterilisiert werden.

In einer bevorzugten Ausgestaltung hat ein erstes der beiden Profilteile ein im wesentlichen U-förmiges Außenwandprofil und verschließt mit seinem Boden das Magazin auf der dem Zangenhauptteil abgewandten Seite. Dieses erste Profilteil ist entlang seiner vom Boden weg zum zweiten der beiden Profilteile hin vorstehenden Seitenwände über die Steckführungen mit dem zweiten Profilteil verbunden. In das erste Profilteil ist ein langgestrecktes, im Querschnitt im wesentlichen U-förmiges Klammerführungsteil eingesetzt, von dessen Bodenteil Seitenteile zum Boden des ersten Profilteils hin abstehen. Das Klammerführungsteil ist in seinen Abmessungen den Wundklammern angepaßt, die mit ihren Schenklen zum Boden des ersten Profilteils hin auf dieses Klammerführungsteil aufgesteckt werden können. Der Bodenteil des Klammerführungsteils und das zweite Profilteil einerseits sowie die Seitenteile des Klammerführungsteils und die Seitenwände des ersten Profilteils andererseits liegen sich jeweils mit Abstand voneinander gegenüber und begrenzen den Führungsschacht. Das Klammerführungsteil und der Boden des ersten Profilteils begrenzen ihrerseits eine Federkammer, in welcher die vorzugsweise als Schraubendruckfeder ausgebildete Klammervorschubfeder platzsparend untergebracht ist. Der Schieber hat in dieser Ausgestaltung einen Ansatz, der durch einen Schlitz des Klammerführungsteils hindurch in die Federkammer eingreift.

Bei dem Klammerführungsteil handelt es sich bevorzugt um ein Blechbiegeteil, welches an seinem der Klammerabgabeöffnung benachbarten Ende einen den Amboß bildenden Lappen trägt. An seinem anderen Ende ist zur Aufnahme der auf den Amboß wirkenden Kippmomente ein über die Höhe des Führungsschachts reichender Abstützvorsprung vorgesehen, der sich an dem zweiten Profilteil abstützt. Zur Befestigung des Blechbiegeteils an dem ersten Profilteil können an Seitenwangen des Blechbiegeteils Federkrallen oder dergleichen vorgesehen sein.

Die Federkammer ist zweckmäßigerweise durch eine Öffnung in einer Stirnwand des ersten Profilteils zugänglich, so daß die Klammervorschubfeder auch nach dem Einbau des Blechbiegeteils eingebaut oder ausgewechselt werden kann. Die Klammervorschubfeder stützt sich an einem Verschlußstück ab, welches in eine zum zweiten Profilteil hin offene Tasche nach dem Einführen der Klammervorschubfeder in den Federschacht in die Tasche eingesteckt wird.

Die Steckführungen sind zweckmäßigerweise als Nut-Rippen-Steckführungen ausgebildet und erstrecken sich vorzugsweise im wesentlichen über die gesamte Länge der Profilteile. Als besonders günstig haben sich zwei Paare von Nuten und Rippen erwiesen, die nach Art einer Schwalbenschwanzführung die beiden Profilteile aneinander führen. Die Führungsflächen der Nuten und Rippen verjüngen sich bevorzugt zur Klammerabgabeöffnung hin und verhindern im Klemmsitz, daß sich die beiden Profilteile beim Gebrauch unbeabsichtigt voneinander lösen.

Die Verrastung des Magazins an dem Zangenhauptteil kann auf vielfältige Weise erfolgen. Betriebsmäßig besonders einfaches Auswechseln ermöglichen Ausgestaltungen, bei welchen eines der Profilteile und das Zangenhauptteil weitere, im wesentlichen in Längsrichtung der Führungsschachts verlaufende Steckführungen aufweisen, die das eine Profilteil quer zum Führungsschacht allseitig fixiert an dem Zangenhauptteil führen. Das Magazin kann längs dieser Steckführungen auf das Zangenhauptteil aufgesteckt werden. In Auftreffrichtung des Biegestempels hingegen wird das Magazin exakt geführt. Zur Fixierung in Richtung der Steckführungen eignet sich insbesondere eine aus einer Rastklinke am Zangenhauptteil und einer Rastaussparung an dem einen Profilteil bestehende Verriegelungsvorrichtung, insbesondere dann, wenn die Rastklinke durch einen griffgünstig am Zangenhauptteil angeordneten Betätigungs-Druckknopf wieder entriegelt werden kann.

Im folgenden soll ein Ausführungsbeispiel der Erfindung anhand von Zeichnungen näher erläutert werden. Es zeigt:
- Fig. 1: einen Längsschnitt durch eine Wundklammerzange;
- Fig. 2: einen Querschnitt durch die Wundklammerzange entlang einer Linie II-II in Fig. 1;
- Fig. 3: einen Querschnitt durch die Wundklammerzange gesehen entlang einer Linie III-III in Fig. 1;
- Fig. 4: einen Querschnitt durch das Magazin der Wundklammerzange, gesehen entlang einer Linie IV-IV in Fig. 1 und
- Fig. 5: eine schematische Darstellung einer mit der Wundklammerzange der Fig. 1 gebogenen Wundklammer.

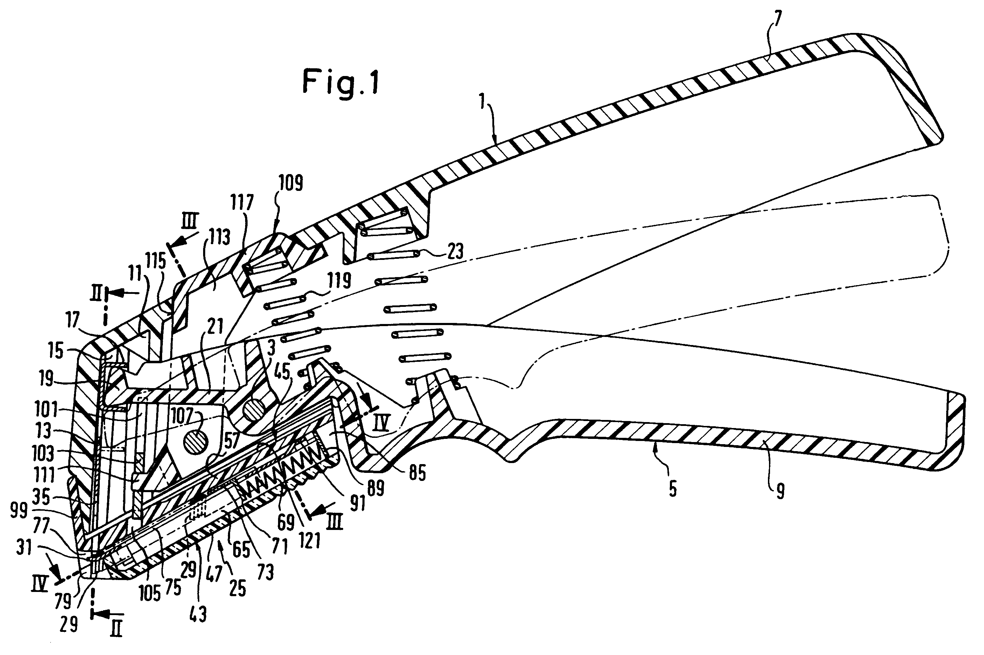

Die in den Fig. 1 bis 4 dargestellte Wundklammerzange umfaßt ein im wesentlichen schalenförmiges Zangenhauptteil 1, an welchem um eine Achse 3 schwenkbar ein Zangengriffteil 5 gelagert ist. Das Zangenhauptteil 1 hat im wesentlichen langgestreckte Form und bildet auf einer Seite der Achse 3 einen Handgriff 7, dem ein Handgriff 9 des Zangengriffteils 5 gegenüberliegt. Auf der dem Handgriff 7 gegenüberliegenden Seiten der Achse 3 bildet das Zangenhauptteil 1 eine Kammer 11, in der quer zur Längsrichtung des Zangenhauptteils 1 ein Biegestempel 13 in einer Führung 15 verschiebbar geführt ist. Der Biegestempel 13 trägt eine Kulisse 17, in die ein Kulissenstein 19 am freien Ende eines dem Handgriff 9 gegenüberliegenden Arms 21 des Zangengriffteils 5 eingreift. Eine zwischen den Handgriffen 7 und 9 geführt eingespannte Schraubendruckfeder 23 hält den Biegestempel 13 in seiner in die Kammer 11 eingezogenen Stellung, aus der er durch Zusammendrücken der Handgriffe 7, 9 ausgeschoben werden kann.

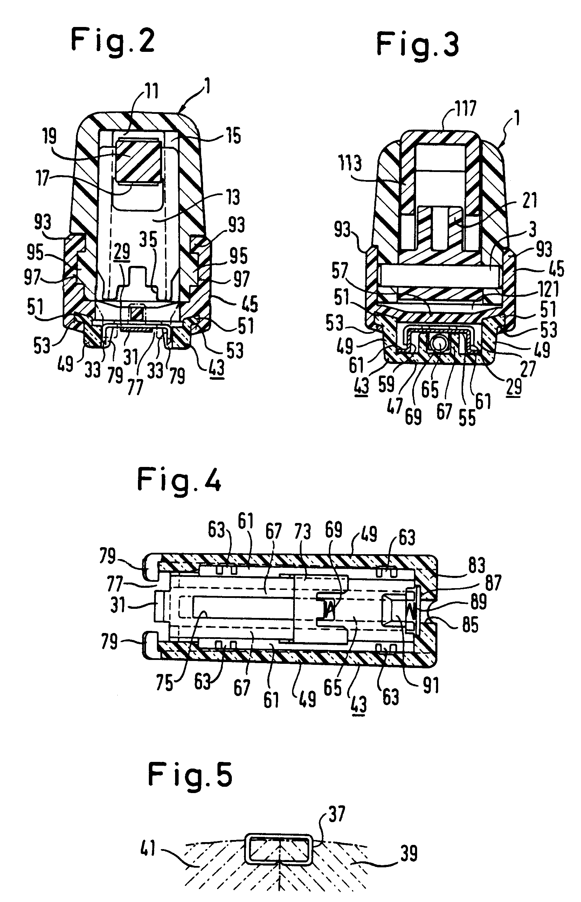

Dem Ausschubende des Biegestemptels 13 gegenüberliegend ist an dem Zangenhauptteil 1 ein Magazin 25 auswechselbar befestigt. Das Magazin 25 umschließt einen langgestreckten, im Querschnitt im wesentlichen U-förmigen Führungsschacht 27 (Fig. 3), in welchem ein Vorrat loser, im wesentlichen U-förmiger Wundklammern 29 in Längsrichtung des Führungsschachts 27 hintereinander untergebracht ist. Das Magazin trägt einen Amboß 31, dessen Breite kleiner ist als die Basislänge der Wundklammern 29, so daß die Schenkel 33 der Wundklammern beiderseits über den Amboß 31 vorstehen und von einer der Amboßbreite unter Berücksichtigung der Dicke der Wundklammern 29 angepaßten Biegeaussparung 35 an der amboßseitigen Stirnseite des Biegestempels 13 zu einem Wundklammerring 37 gebogen werden, der die Wundhälften 39, 41 zusammenklammert, wie dies in Fig. 5 dargestellt ist.

Das Magazin ist als Einheit auswechselbar an dem Zangenhauptteil 1 gehalten. Es umfaßt zwei langgestreckte Profilteile 43, 45, die den Führungsschacht 27 umschließen. Das Profilteil 43 hat ein im Querschnitt im wesentlichen U-förmiges Außenwandprofil mit einer Bodenwand 47, von der zwei Seitenwände 49 zum Profilteil 45 hin abstehen. An den freien Längsrändern der Seitenwände 49 sind in Längsrichtung des Führungskanals 27 verlaufende, voneinander weg abstehende Längsrippen 51 vorgesehen, die nach Art einer Schwalbenschwanzführung in komplementäre, am anderen Profilteil 45 vorgesehene Längsnuten 53 eingreifen. Die Längsrippen 51 und Längsnuten 53 bilden Steckführungen, längs der das Profilteil 43 an dem Profilteil 45 verschiebbar geführt ist. Quer zur Längsrichtung fixieren diese Steckführungen die beiden Profilteile 43, 45 aneinander.

In das Profilteil 43 ist ein im wesentlichen U-förmiges, als Blechbiegeteil ausgebildetes Klammerführungsteil 55 eingesetzt, dessen Außenabmessungen den Innenabmessungen der Wundklammern 29 angepaßt ist. Das Klammerführungsteil 55 begrenzt zusammen mit einer die Nuten 53 verbindenden Querwand 57 des Profilteils 45 einerseits und den Seitenwänden 49 des Profilteils 43 andererseits den Führungsschacht 27. An den freien Längsrändern der Seitenteile 59 des Klammerführungsteils 55 sind voneinander weg abstehende Wangen 61 angeformt, aus welchen, wie am besten Fig. 4 zeigt, Zungen 63 freigestanzt sind, die mit den Seitenwänden 49 des Profilteils 43 verkrallt sind und das Klammerführungsteil 55 an dem Profilteil 43 halten.

Der vom Inneren des Klammerführungsteils 55 und der Bodenwand 47 umschlossene Bereich bildet eine Federkammer 65, in welcher durch Rippen 67 geführt eine längs des Führungsschachts 27 wirkende Schraubendruckfeder 69 angeordnet ist. Die Feder 69 stützt sich an einem Lappen 71 eines Schiebers 73 ab, der in dem Führungsschacht 27 längsverschiebbar auf dem Klammerführungsteil 55 reitet. Der Lappen 71 greift durch einen Längsschlitz 75 des Klammerführungsteils 55 hindurch in die Federkammer 65.

Die Feder 69 drückt die Wundklammern 29 in Richtung einer den Amboß 31 ringförmig umgebenden Abgabeöffnung 77. Vor der Abgabeöffnung 77 sind von den Seitenwänden 49 zum Amboß hin vorstehende Nasen 79 angeordnet, die den Ausschubweg der Klammern 29 begrenzen.

Das dem Amboß 31 gegenüberliegende Ende des Profilteils 43 ist durch eine Stirnwand 83 verschlossen, welche eine mit der Federkammer 65 fluchtende Einführöffnung 85 für die Feder 69 enthält. In einer Tasche 87 der Stirnwand 83 sitzt ein bei abgenommenem Profilstück 45 einsteckbares Verschlußstück 89, an welchem sich die Feder 69 mit ihrem dem Schieber 73 abgewandten Ende abstützt. Das Klammerführungsteil 55 hat an seinem dem Amboß 31 abgewandten Ende zur Querwand 57 des Profilteils 45 vorstehende Anprägung 91, über die sich das Klammerführungsteil 55 bei der Druckbelastung des Ambosses 31 kippsicher abstützt. Das Magazin 25 wird wie folgt zusammengebaut:

Das Klammerführungsteil 55 wird zunächst in der dargestellten Weise in das Profilteil 43 eingesetzt, wobei sich die Zungen 63 in den Seitenwänden 49 verkrallen. Dann werden die Wundklammern 29 auf das klammerführungsteil 55 aufgelegt, wobei sukzessive das Profilteil 45 auf das Profilteil 43 aufgesteckt wird, um das Herausfallen und Verschieben der bereits eingelegten Klammern zu verhindern. Es wird der Schieber 73 aufgesetzt und das Profilteil 45 zur Fixierung des Schiebers 73 bis nahe an die Tasche 87 herangeschoben. Jetzt wird die Feder 69 durch die Einführöffnung 85 eingeführt und mit einem Werkzeug, beispielsweise einem Schraubenzieher oder dergleichen bis vor die Tasche 87 gespannt, worauf das Verschlußstück 89 in die Tasche 87 eingesetzt wird. Abschließend werden die Profilteile 43, 45 fertig ineinandergeschoben. Die Führungsflächen der Längsrippen 51 und der Nuten 53 verlaufen, wie in den Fig. 2 bis 4 angedeutet ist, aufeinander zu, so daß das Profilteil 43 im Klemmsitz zur Abgabeöffnung 77 hin fixiert ist.

Das Profilteil 45 hält das Magazin 25 an dem Zangenhauptteil 1. Auf der zum Zangenhauptteil 1 weisenden Seite der Querwand 57 trägt das Profilteil 45 Wangen 93, die das Zangenhauptteil 1 zwischen sich einschließen. An dem Zangenhauptteil 1 sind in Längsrichtung des Führungsschachts 27 verlaufende Rippen 95 vorgesehen, die in komplementäre Längsnuten 97 der Wangen 93 eingreifen. Das Profilteil 45 und damit das Magazin 25 läßt sich somit auf das Zangenhauptteil 1 aufstecken. Der Klammerabgabeöffnung 77 benachbart sind die Wangen 93 durch eine Stirnwand 99 miteinander verbunden, die den Einschubweg des Magazins 25 begrenzt.

Zur Verriegelung des Magazins 25 an den Zangenhauptteil 1 ist in Nuten 101 der Kammer 11 eine plattenförmige Klinke 103 verschiebbar geführt, die in eine Öffnung 105 der Querwand 57 eingreift und im Eingriffszustand das Magazin verrastet. Ein an einer Achse 107 schwenkbar an dem Zangenhauptteil 1 gelagerter, doppelarmiger Bedienungshebel 109 greift mit seinem einen Arm 111 in die Klinke 103 und trägt an seinem anderen Arm 113 einen durch eine Öffnung 115 auf der dem Magazin 25 abgewandten Seite des Zangenhauptteils 1 austretenden Betätigungs-Druckknopf 117. Eine zwischen dem Arm 113 und dem Handgriff 9 des Zangengriffteils 5 geführt eingespannte Schraubendruckfeder 119 spannt die Klinke 103 in Verriegelungsrichtung vor und unterstützt, da sie wirkungsmäßig der Feder 23 parallel geschaltet ist, die Öffnungsbewegung der Zange, so daß die Feder 23 schwächer dimensioniert sein kann.

Das Magazin 25 wird von der dem Handgriff 7 abgewandten Ende des Zangenhauptteils 1 auf die Längsrippen 95 aufgesteckt. Die der Klinke 103 zugewandte Außenfläche 121 der Querwand 57 ist in Form einer Einführschräge ausgebildet, über die die Klinke 103 in die Öffnuing 105 selbttätig einschnappt. Zum Lösen des Magazins 25 wird der Druckknopf 117 gedrückt, womit das Magazin frei abgezogen werden kann.

Das Zangenhauptteil 1, das Zangengriffteil 5, der Hebel 109 sowie die Profilteile 43 und 45 bestehen aus spritzgußgeformtem, sterilisierbarem Kunststoff. Zweckmäßigerweise besteht das Profilformteil 43 aus einem transparenten Kunststoffmaterial, um von außen her erkennen zu können, wieviele Wundklammern das Magazin noch enthält. Der Biegestempel 13, das Führungsprofilteil 55 und die Klinke 103 sind vorzugsweise aus nichtrostendem Stahl gefertigt.

## Patentansprüche

1. Wundklammerzange,
mit einem Zangenhauptteil (1),
mit einem an dem Zangenhauptteil (1) gehaltenen Magazin (25), welches zwei Profilteile (43, 45), die gemeinsam einen langgestreckten, im Querschnitt im wesentlichen U-förmigen Führungsschacht (27) für einen Vorrat von im wesentlichen U-förmigen Wundklammern (29) bilden, einen in dem Führungsschacht (27) verschiebbaren Schieber (73) sowie eine den Schieber (73) auf eine Klammerabgabeöffnung (77) des Führungsschachts (27) zu vorspannende Klammervorschubfeder (69) aufweist,
mit vor der Klammerabgabeöffnung (77) angeordneten, den Klammerausschubweg begrenzenden Anschlagmitteln (79), mit einem Klammerbiegemechanismus, welcher einen im Bereich der Klammerabgabeöffnung (77) des Magazins (25) angeordneten Amboß (31) und einen relativ zu dem Amboß (31) beweglich an dem Zangenhauptteil (1) geführten Biegestempel (13) aufweist
und mit einem schwenkbar an dem Zangenhauptteil (1) gelagerten Zangengriffteil (5) zur Betätigung des Klammerbiegemechanismus,
**dadurch gekennzeichnet,** daß die beiden Profilteile (43, 45) des Magazins (25) in Längsrichtung des Führungsschachts (27) verlaufende Steckführungen (51, 53) aufweisen, die die Profilteile (43, 45) quer zum Führungsschacht (27) aneinander allseitig fixiert führen,
daß die den Klammerausschubweg begrenzenden Anschlagmittel zwei beiderseits der Klammerabgabeöffnung (77) vor dieser an dem Magazin (25) angeordnete Anschläge (79) aufweisen, daß auch der Amboß (31) an dem Magazin (25) angeordnet ist und die Klammerabgabeöffnung (77) den Amboß (31) umbigt und daß das Magazin (25) zusammen mit dem Schieber (73) und der Klammervorschubfeder (69) als Einheit lösbar an dem Zangenhauptteil (1) befestigt ist.

2. Wundklammerzange nach Anspruch 1, dadurch gekennzeichnet, daß ein erstes (43) der Profilteile ein im wesentlichen U-förmiges Außenwandprofil hat und mit seinem Boden (47) das Magazin (25) auf der dem Zangenhauptteil (1) abgewandten Seite verschließt sowie entlang seiner vom Boden (47) weg zum zweiten (45) der Profilteile vorstehenden Seitenwände (49) über die Steckführungen (51, 53) mit dem zweiten Profilteil (45) verbunden ist, daß in das erste Profilteil (43) ein langgestrecktes, im Querschnitt im wesentlichen U-förmiges Klammerführungsteil (55) eingesetzt ist, von dessen Bodenteil Seitenteile (59) zum Boden (47) des ersten Profilteils (43) abstehen, wobei sich der Bodenteil und das zweite Profilteil (43) einerseits sowie die Seitenteile (59) und die Seitenwände (49) des ersten Profilteils (43) andererseits jeweils mit Abstand voneinander gegenüberliegen und den Führungsschacht (27) begrenzen,
daß die Klammervorschubfeder (69) in einer durch das Klammerführungsteil (55) und den Boden (47) des ersten Profilteils (43) begrenzten Federkammer (65) angeordnet ist
und daß das Klammerführungsteil (55) einen in Längsrichtung verlaufenden Schlitz (75) aufweist, durch den hindurch ein Ansatz (71) des Schiebers (73) in die Federkammer (65) eingreift.

3. Wundklammerzange nach Anspruch 2, dadurch gekennzeichnet, daß das Klammerführungsteil (55) als Blechbiegeteil ausgebildet ist und an seinem der Klammerabgabeöffnung (77) benachbarten Ende einen den Amboß (31) bildenden Lappen und an seinem anderen Ende einen zum zweiten Profilteil (45) vorspringenden Abstützvorsprung (91) trägt.

4. Wundklammerzange nach Anspruch 3, dadurch gekennzeichnet, daß die dem Bodenteil abgewandten Längsränder der Seitenteile (59) des Klammerführungsteils (55) an den Seitenwänden (49) des ersten Profilteils (43) abgestützte Seitenwangen (61) haben, aus welchen Federkrallen (63) freigestanzt sind.

5. Wundklammerzange nach einem der Ansprüche 2 bis 4, dadurch gekennzeichnet, daß das der Klammerabgabeöffnung (77) abgewandte Ende des Führungsschachts (27) durch eine Stirnwand (83) des ersten Profilteils (43) verschlossen ist, daß die Stirnwand (83) eine Öffnung (85) zum Einführen der Klammervorschubfeder (69) in die Federkammer (65) aufweist und daß im Bereich der Stirnwand (83) eine zum zweiten Profilteil (45) hin offene Tasche (87) zum Einstecken eines die Einführöffnung (85) verschließenden Verschlußstücks (89) vorgesehen ist, an dem sich die Klammervorschubfeder (69) abstützt.

6. Wundklammerzange nach einem der Ansprüche 1 bis 5, dadurch gekennzeichnet, daß die Steckführungen (51, 53) als im wesentlichen über die gesamte Länge eines (45) der Profilteile sich erstreckende Nuten (53) ausgebildet sind, denen in Längsrichtung sich erstreckende Rippen (51) des anderen Profilteils (43) zugeordnet sind.

7. Wundklammerzange nach Anspruch 6, dadurch gekennzeichnet, daß zwei zueinander entgegengerichtet vorspringende Paare von Nuten (53) und Rippen (51) vorgesehen sind, deren Führungsflächen in Längsrichtung zur Klammerabgabeöffnung hin aufeinander zu verlaufen.

8. Wundklammerzange nach einem der Ansprüche 1 bis 7, dadurch gekennzeichnet, daß eines der Profilteile (45) und das Zangenhauptteil (1) weitere, im wesentlichen in Längsrichtung des Führungsschachts (27) verlaufende Steckführungen (95, 97) aufweisen, die das eine Profilteil (45) quer zum Führungsschacht (27) allseitig fixiert an dem Zangenhauptteil (1) führen.

9. Wundklammerzange nach Anspruch 8, dadurch gekennzeichnet, daß in dem einen Profilteil (45) eine Rastaussparung (105) und an dem Zangenhauptteil (1) eine Rastklinke (103) beweglich geführt ist, die von einer Rastfeder (119) in Richtung des Eingriffs in die Rastaussparung (105) vorgespannt ist.

10. Wundklammerzange nach Anspruch 9, dadurch gekennzeichnet, daß in dem Zangenhauptteil (1) parallelachsig zum Zangengriffteil (5) ein doppelarmiger Hebel (109) gelagert ist, der mit seinem einen Arm (111) mit der Rastklinke (103) verbunden ist und dessen anderer Arm (113) einen durch eine Öffnung (115) des Zangenhauptteils (1) austretenden Betätigungs-Druckknopf (117) bildet.

11. Wundklammerzange nach Anspruch 10, dadurch gekennzeichnet, daß die Rastfeder (119) zwischen dem anderen Arm (113) und dem Zangengriffteil (5) eingespannt ist und das Zangengriffteil (5) in Zangenöffnungsrichtung belastet.

## Claims

1. A surgical stapling gun, comprising a main gun part (1) and with, supported on the main gun part (1), a magazine (25) comprising two profiled parts (43, 45) which jointly form an elongate cross-sectionally substantially U-shaped guide shaft (27) for a supply of substantially U-shaped wound staples (29), a slider (73) displaceable in the guide shaft (27) and a staple feed spring (69) which pre-tensions the slider (73) towards a staple delivery aperture (77) in the guide shaft (27), and with, disposed in front of the staple delivery aperture (77) and defining the staple ejection path, abutment means (79) and with a staple bending mechanism comprising an anvil (31) disposed in the region of the staple delivery aperture (77) of the magazine (25) and a bending stamp (13) guided on the main gun part (1) for movement in relation to the anvil (31) and with, pivotally mounted on the main gun part (1) a gun handle part (5) for actuating the staple bending mechanism, characterised in that the two profiled parts (43, 45) of the magazine (25) have, extending in the longitudinal direction of the guide shaft (27), push-in guides (51, 53) which guide the profiled parts (43, 45) so that they are fixed to each other all round cross-wise to the guide shaft (27), and in that the abutment means defining the staple ejection path comprise two abutments (79) disposed on the magazine (25) and in front of and on both sides of the staple ejection aperture (77) and in that also the anvil (31) is disposed on the magazine (27) and in that the staple ejection aperture (77) surrounds the anvil (31) and in that the magazine (25), together with the slider (73) and the staple feed spring (69), is as one unit separably mounted on the main gun part (1).

2. A surgical stapling gun according to Claim 1, characterised in that a first (43) of the profiled members has a substantially U-shaped outer wall profile while its bottom (47) encloses the magazine (25) on the side remote from the main gun part (1) and is along its side walls (49) which project away from the bottom (47) towards the second (45) of the profiled parts connected to the second profiled part (45) by means of the push-in guides (51, 53), and in that there is inserted into the first profiled part (43) an elongate cross-sectionally substantially U-shaped staple guide part (55) from the bottom part of which lateral parts (59) project towards the bottom (47) of the first profiled part (43), the bottom part and the second profiled part (43) on the one hand and the lateral parts (59) and side walls (49) of the first profiled part (43) on the other being respectively spaced apart from and opposite one another and defining the guide shaft (27) and in that the staple feed spring (69) is disposed in a spring chamber (65) defined by the staple guide part (55) and the bottom (47) of the first profiled part (43) and in that the staple guide part (55) has extending in a longitudinal direction a slot (75) through which a projection (71) on the slider (73) engages the spring chamber (65).

3. A surgical stapling gun according to Claim 2, characterised in that the staple guide part (55) is constructed as a bent sheet metal part and has at its end adjacent the staple delivery aperture (77) a tang which forms the anvil (31) and has at its other end and projecting towards the second profiled part (45) a bracing projection (91).

4. A surgical stapling gun according to Claim 3, characterised in that the longitudinal edges of the side parts (59) of the staple guide part (55) which are directed away from the bottom part have braced against the side walls (49) of the first profiled part (43) lateral cheeks (61) out of which spring claws (63) are stamped.

5. A surgical stapling gun according to one of Claims 2 to 4, characterised in that the end of the guide shaft (27) which is remote from the staple delivery aperture (77) is closed by an end wall (83) of the first profiled member (43) and in that the end wall (83) comprises an aperture (85) for insertion of the staple feed spring (69) into the spring chamber (65) and in that in the region of the end wall (83) there is, open towards the second profiled part (45), a pocket (87) for insertion of a closure member (89) which occludes the insertion aperture (85) and on which the staple feed spring (69) is biased.

6. A surgical stapling gun according to one of Claims 1 to 5, characterised in that the push-in guides (51, 53) are constructed as grooves (53) extending over substantially the entire length of one (45) of the profiled parts and with which longitudinally extending ribs (51) on the other profiled part (43) are associated.

7. A surgical stapling gun according to Claim 6, characterised in that two pairs of grooves (53) and ribs (51) are provided which project towards one another and the guide surfaces of which extend towards one another in the longitudinal direction towards the staple delivery aperture.

8. A surgical stapling gun according to one of Claims 1 to 7, characterised in that one of the profiled parts (45) and the main part (1) of the gun has further push-in guides (95, 97) extending substantially in the longitudinal direction of the guide shaft (27) and which guide one profiled part (45) cross-wise to the filling shaft (27) so that it is fixed all round on the main part (1) of the gun.

9. A surgical stapling gun according to Claim 8, characterised in that a catch recess (105) is provided in one profiled part (45) while movably guided on the main part (1) of the gun is a detent (103) which is pre-tensioned by a ratchet spring (119) in the direction of engagement into the catch recess (105).

10. A surgical stapling gun according to Claim 9, characterised in that mounted in the main part (1) of the gun, its axis parallel with the gun grip (5), is a double-armed lever (109) of which one arm (111) is connected to the detent (103) while its other arm (113) forms an actuating push-button (117) emerging through an aperture (115) in the main part (1) of the gun.

11. A surgical stapling gun according to Claim 10, characterised in that the catch spring (119) is clamped between the other arm (113) and the grip part (5) of the gun and loads the said grip part (5) of the gun in the direction of gun opening.

## Revendications

1. Agrafeuse pour blessure comportant une partie principale (1), comportant un magasin (25), fixé sur la partie principale (1), lequel présente deux éléments profilés (43, 45) qui forment conjointement un compartiment de guidage (27) de section transversale sensiblement en U pour une réserve d'agrafes pour blessure (29) sensiblement en U, un coulisseau (73) déplaçable dans le compartiment de guidage (27) ainsi qu'un ressort d'avance d'agrafes (69) à précontraindre sur une ouverture de distribution d'agrafes (77) du compartiment de guidage (27), comportant des moyens de butée (79) situés en amont de l'ouverture de distribution d'agrafes (77), limitant le parcours d'éjection des agrafes, comportant un mécanisme de pliage d'agrafes, qui présente une enclume (31) située dans la zone de l'ouverture de distribution d'agrafes (77) du magasin (25) et un poinçon de pliage (13), monté mobile par rapport à l'enclume (31) sur la partie principale (1) de l'agrafeuse,et comportant un élément de poignée d'agrafeuse (5) monté pivotant sur la partie principale (1), pour l'actionnement du mécanisme de pliage des agrafes, caractérisée en ce que les deux éléments profilés (43, 45) du magasin (25) présentent des organes de guidage à emboîtement (51, 53), s'étendant dans la direction longitudinale du compartiment de guidage (27), qui guident les éléments profilés (43, 45), transversalement au compartiment de guidage (27), fixés de tous côtés l'un contre l'autre, en ce que les moyens de butée, limitant le parcours d'éjection des agrafes, comportent deux butées (79) placées des deux côtés de l'ouverture de distribution d'agrafes (77), en amont de celle-ci, sur le magasin (25), en ce que l'enclume (31) se situe aussi sur le magasin (25) et l'ouverture de distribution d'agrafes (77) entoure l'enclume (31) et en ce que le magasin (25), formant une unité avec le coulisseau (73) et le ressort d'avance d'agrafes (69), est fixé de manière amovible sur la partie principale (1) de l'agrafeuse.

2. Agrafeuse pour blessure selon la revendication 1, caractérisée en ce qu'un premier élément (43) des éléments profilés présente un profil de paroi extérieure sensiblement en U et ferme, avec son fond (47), le magasin (25), sur le côté opposé à l'élément principal (1) et est relié avec le second élément profilé (45), le long de ses parois latérales (49) dépassant du fond (47) vers le second (45) des éléments profilés, par l'intermédiaire des organes de guidage par emboîtement (51, 53), en ce que dans le premier élément profilé (43) est insérée une pièce de guidage d'agrafes (55) allongée, de section transversale sensiblement en U, dont des parties latérales (59) dépassent de sa partie de fond vers le fond (47) du premier élément profilé (43), la partie de fond et le second élément profilé (43) d'une part ainsi que les parties latérales (59) et les parois latérales (49) du premier élément profilé (43) d'autre part, se faisant face les unes les autres à une certaine distance et limitant le compartiment de guidage (27), en ce que le ressort d'avance d'agrafes (69) est placé dans une chambre de ressort (65) limitée par la pièce de guidage d'agrafes (55) et le fond (47) du premier élément profilé (43) et en ce que la pièce de guidage d'agrafes (55) présente une fente (75) s'étendant dans la direction longitudinale à travers laquelle un appendice (71) du coulisseau (73) s'engage dans la chambre de ressort (65).

3. Agrafeuse pour blessure selon la revendication 2, caractérisée en ce que la pièce de guidage d'agrafes (55) est une pièce cintrée en tôle et porte, à son extrémité voisine de l'ouverture de distribution d'agrafes (77), une patte formant l'enclume (31) et à son autre extrémité, un saillie d'appui (91) dépassant du second élément profilé (45).

4. Agrafeuse pour blessure selon la revendication 3, caractérisée en ce que les bords longitudinaux, tournés à l'opposé de la partie de fond, des parties latérales (59) de la pièce de guidage d'agrafes (55), ont des flasques latéraux (61) prenant appui contre les parois latérales (49) du premier élément profilé (43), dans lesquels sont découpées des griffes élastiques (63).

5. Agrafeuse pour blessure selon l'une des revendications 2 à 4, caractérisée en ce que l'extrémité, tournée à l'opposé de l'ouverture de distribution d'agrafes (77), du compartiment de guidage (27), est fermée par une paroi frontale (83) du premier élément profilé (43), en ce que la paroi frontale (83) présente une ouverture (85) destinée a l'introduction du ressort d'avance d'agrafes (69) dans la chambre de ressort (65) et en ce que dans la zone de la paroi frontale (83) il est prévu une poche (87), ouverte vers le second élément profilé (45), pour l'insertion d'une pièce de fermeture (89), fermant l'ouverture d'introduction (85), contre laquelle prend appui le ressort d'avance d'agrafes (69).

6. Agrafeuse pour blessure selon l'une des revendications 1 à 5, caractérisée en ce que les organes de guidage par emboîtement (51, 53) sont conçus sous la forme de rainures (53), s'étendant sensiblement sur toute la longueur de l'un (45) des éléments profilés, auxquelles sont associées des nervures (51), s'étendant dans la direction longitudinale, de l'autre élément profilé (43).

7. Agrafeuse pour blessure selon la revendication 6, caractérisée en ce qu'il est prévu deux paires saillantes, dirigées à l'opposé l'une de l'autre, de rainures (53) et de nervures (51) dont les surfaces de guidage s'étendent l'une vers l'autre, dans la direction longitudinale, vers l'ouverture de distribution d'agrafes.

8. Agrafeuse pour blessure selon l'une des revendications 1 à 7, caractérisée en ce que l'un des éléments profilés (45) et la partie principale (1) de l'agrafeuse comportent d'autres organes de guidage par emboîtement (95, 97), s'étendant sensiblement dans la direction longitudinale du compartiment de guidage (27), lesquels guident un élément profilé (45), transversalement au compartiment de guidage (27), fixé de tous côtés sur la partie principale (1) de l'agrafeuse.

9. Agrafeuse pour blessure selon la revendication 8, caractérisée en ce que dans un élément profilé (45) est prévue une découpe d'encliquetage (105) et sur la partie principale (1) de l'agrafeuse est monté mobile un cliquet (103) qui est précontraint par un ressort d'encliquetage (119), dans le sens de l'engagement dans la découpe d'encliquetage (105).

10. Agrafeuse pour blessure selon la revendication 9, caractérisée en ce qu'il est monté dans la partie principale (1) de l'agrafeuse, parallèlement à l'axe de l'élément de poignée (5) de l'agrafeuse, un levier (109) à deux bras qui par l'un de ses bras (111) est relié au cliquet (103) et dont l'autre bras (113) forme un boutonpoussoir d'actionnement (117), sortant par une ouverture (115) de la partie principale (1) de l'agrafeuse.

11. Agrafeuse pour blessure selon la revendication 10, caractérisée en ce que le ressort d'encliquetage (119) est serré entre l'autre bras (113) et l'élément de poignée (5) de l'agrafeuse et agit sur celuici dans le sens de l'ouverture de l'agrafeuse.
